# EUROPÄISCHE PATENTSCHRIFT

(11) **EP 3 801 403 B1**
(45) Veröffentlichungstag und Bekanntmachung des Hinweises auf die Patenterteilung: **18.11.2026**
(21) Anmeldenummer: 19728545.5
(22) Anmeldetag: 21.05.2019
(51) Int. Cl.: A61F 2/80, A61F 2/68, B06B 1/00, B06B 1/16, B06B 3/00

(54) **STIMULIERUNGSEINRICHTUNG UND PROTHESENVORRICHTUNG MIT ZUMINDEST EINER STIMULIERUNGSEINRICHTUNG ZUM STIMULIEREN VON NERVENZELLENENDEN SOWIE DIE VERWENDUNG EINES VIBRATIONSERZEUGERS ZUM SCHWINGUNGSENTKOPPELTEN STIMULIEREN VON NERVENZELLENENDEN**
STIMULATION DEVICE AND PROSTHETIC DEVICE WITH AT LEAST ONE STIMULATOR FOR THE STIMULATION OF NEURAL CELLS ENDS AS WELL AS THE USE OF A VIBRATION GENERATOR FOR A VIBRATION-DECOUPLED STIMULATION OF NEURAL CELLS ENDS
DISPOSITIF DE STIMULATION ET DISPOSITIF PROSTHÉTIQUE DOTÉ D'AU MOINS UN DISPOSITIF DE STIMULATION DESTINÉ À LA STIMULATION DES EXTRÉMITÉS DES CELLULES NERVEUSES AINSI QUE'UTILISATION D'UN GÉNÉRATEUR DE VIBRATIONS DESTINÉE À LA STIMULATION ISOLÉE DES VIBRATIONS DES EXTRÉMITÉS DES CELLULES NERVEUSES

(30) Priorität: 30.05.2018 EP 18175172
(43) Veröffentlichungstag der Anmeldung: 14.04.2021
(73) Patentinhaber: Saphenus Medical Technology GmbH, 2500 Baden (AT)
(72) Erfinder: BRANDSTÄTTER, Martin, 1090 Wien (AT); LANG, Bernhard, 1220 Wien (AT); PITSCHL, Aaron, 1220 Wien (AT)
(74) Vertreter: Neuhold, Alfred
(86) Internationale Anmeldenummer: PCT/IB2019/054191
(87) Internationale Veröffentlichungsnummer: WO 2019/229587

(56) Entgegenhaltungen:
- EP-A1- 3 125 574
- WO-A1-2011/117387
- WO-A1-98/25552
- CN-A- 106 691 814
- KR-A- 20120 092 226
- US-A1- 2009 024 062
- US-A1- 2012 143 351

## Beschreibung

Die Erfindung betrifft eine Prothesenvorrichtung mit zumindest einer Stimulierungseinrichtung zum Stimulieren von Nervenzellenenden nach dem Oberbegriff des unabhängigen Anspruchs.

Am 8.Juni 2015 wurde durch die Tageszeitung derStandard.at unter Wissenschaft > Mensch ein Artikel publiziert, der Auskunft über eine Entwicklung von Professor Hubert Egger, einem der weltweit führenden Prothesenforschern gibt. Es wird über die erste Beinprothese berichtet, die «mitfühlt». Ausführlich wird dort dargelegt, dass bei einem beinamputierten Patienten abgetrennte Nerven reaktiviert wurden und in ein Hautareal am Beinstumpf verlegt wurden. Da dieses Hautareal anschliessend die reinnervierten Nervenzellenenden aufweist, wird diese Hautareal besonders empfindlich. Die Nervenenden wurden dabei so verlegt, dass in diesem Hautareal durch die dorthin verlegten Nervenenden der ehemalige Fuss mit seinen physionomietypischen Nervenarealabschnitten abgebildet wurde. Mit anderen Worte ist ein physionomietypischer Nervenarealabschnitt ein natürlicher Nervenarealabschnitt, welcher an die Oberfläche eines dafür eigentlich untypischen Hautareals verlegt wird. Der Patient fühlte daher an diesem Hautareal nicht (nur) wie üblich die dort befindliche Haut(oberfläche), sondern die reinnervierten Nervenzellenenden und somit quasi seine Fusssohle, die ja infolge Amputation des Beins gar nicht mehr vorhanden ist.

Die bei der vorgestellten Lösung verwendeten Stimulatoren waren so ausgebildet, dass sie direkt im Schaft der Prothese ein Vibrationssignal abgaben, um über die Schaftwand per Vibrationssignal dem Patienten anzuzeigen, dass nun genau dieser Sohlenabschnitt der Kunstsohle mit Druck belastet wurde. Die bei diesem Aufbau als Stimulatoren verwendeten Vibratoren erzeugten dabei ein kurzzeitiges Vibrieren im Schaft der Prothese, sobald der mit dem jeweiligen Vibrator gekoppelte Drucksensor am entsprechenden Sohlenareal der Kunstfusssohle ein Drucksignal aufnahm.

Nachteilig bei diesem Aufbau ist, dass das Vibrieren der Vibratoren auf den Prothesenschaft übertragen wird und sich somit zum Teil auch über die Schaftoberfläche verteilt. Das führt dazu, das die Präzision der angestrebten punktförmigen Anzeige schwindet und ausserdem das Schaftmaterial wie ein Resonanzkörper wirkt und demzufolge auch eine Geräuschentwicklung wahrnehmbar ist, die für einen Benutzer und für seine Umgebung als störend wahrgenommen werden kann.

Die CN 106691814 A offenbart einen Vibrationsmassagestab mit einem rotierbaren Stab als Vibrationserzeuger, welcher mit einem Motor angetrieben wird und welcher das Gehäuse des Vibrationsmassagestabs in Vibration versetzt. Der Motor ist in einer Griffeinheit des Vibrationsmassagestabs angeordnet. Zwischen dem Motor und dem rotierenden Stab ist ein Kopplungselement zur Übertragung der Antriebsbewegung auf den rotierbaren Stab angeordnet. Weiters ist eine separate Dämpfungseinheit an der Griffeinheit angeordnet, um die Übertragung der Vibrationen des Gehäuses auf die Griffeinheit zu unterdrücken.

Nachteilig an dieser Lösung ist, dass eine zusätzliche Dämpfungseinheit zur Minderung der Vibrationsübertragung auf die Griffeinheit des Vibrationsmassagestabs notwendig ist. Gattungsgemässe Stimulierungseinrichtungen sind auch in der KR 2012 092226 A1 und der EP 3 125 574 A1 offenbart.

Die WO 2009/014644 A1 kann als nächstliegender Stand der Technik angesehen werden und offenbart eine Orthopädievorrichtung bzw. eine Prothesenvorrichtung für eine menschliche Extremität mit einem Feedbacksystem zum Alarmieren eines Benutzers, wobei die Alarmierung des Benutzers, beispielsweise bei einer Fehlstellung des menschlichen Gelenks, mithilfe eines Vibrationserzeugers erfolgt. Damit wird der Benutzer mithilfe der Vibration des Vibrationserzeugers bei jeder Fehlstellung des Gelenks konditioniert, wobei die Vibrationen entweder im Bereich des Gelenks oder an einen anderen Körperteil übermittelt wird.

Nachteilig an dieser bekannten Lösung ist, dass die Vibrationserzeuger direkt innerhalb der Orthopädievorrichtung bzw. der Prothesenvorrichtung angeordnet sind und die Vibrationen des Vibrationserzeugers auf bzw. direkt in der Orthopädievorrichtung bzw. der Prothesenvorrichtung wirken. Auch hier kommt es somit zu Resonanzerscheinungen und Geräuschentwicklung.

Es ist die Aufgabe der vorliegenden Erfindung einen oder mehrere Nachteile des Standes der Technik zu beheben. Insbesondere soll eine Prothesenvorrichtung mit zumindest einer Stimulierungseinrichtung zum Stimulieren von Nervenzellenenden, welche eine Geräuschunterdrückung während dem Stimulieren von Nervenzellen ermöglicht und somit den Tragekomfort für den Träger der Prothese verbessert sowie für den angestrebten Zweck nämlich der Signalübertragung an innervierte Nervenenden besser geeignet ist. Die Aufgabe wird durch die in den unabhängigen Ansprüchen definierten Vorrichtungen gelöst. Vorteilhafte Weiterbildungen sind in den Figuren, der Beschreibung und insbesondere in den abhängigen Patentansprüchen dargelegt.

Die Erfindung betrifft eine Prothesenvorrichtung mit zumindest einer Stimulierungseinrichtung zur Informationsabgabe an Nervenzellenenden mit einem Prothesenschaft, wobei die zumindest eine Stimulierungseinrichtung zumindest einen Vibrationserzeuger umfasst. Der zumindest eine Vibrationserzeuger ist über zumindest ein Entkopplungselement mit dem Prothesenschaft lagefest verbunden. Damit wird eine Übertragung der Vibration bzw. Schwingung vom zumindest einen Vibrationserzeuger auf den Prothesenschaft unterbunden, wodurch Anwender bzw. der Patient der Prothesenvorrichtung die Vibration bzw. Schwingung an dem jeweiligen zu stimulierenden Nervenzellenende spüren kann und gleichzeitig keine Irritierung durch eine Geräuschentstehung am Prothesenschaft erfährt. Damit wird der Tragekomfort für den Prothesenträger verbessert.

Bevorzugt ist die zumindest eine Stimulierungseinrichtung die hier vorliegend beschriebene Stimulierungseinrichtung, wodurch eine besonders effektive Geräuschunterdrückung beim Stimulieren der Nervenzellenenden erzielt wird.

Insbesondere umfasst die Informationsabgabe an die Nervenzellenenden ein Stimulieren von Nervenzellenenden, insbesondere physionomietypischen Nervenarealabschnitten, wodurch die Nervenzellenenden trainiert werden können.

Vorzugsweise weist das zumindest eine Entkopplungselement einen Befestigungsabschnitt zum Befestigen des Entkopplungselements an dem Prothesenschaft auf. Damit lässt sich der zumindest eine Vibrationserzeuger stabil am Prothesenschaft halten. Typischerweise befindet sich der Befestigungsabschnitt an einem Ende des zumindest einen Entkopplungselements, sodass sich ein effektives Entkoppeln beim Vibrieren bzw. beim Schwingen gewährleisten lässt und gleichzeitig ein effektives Stimulieren der Nervenzellendenden möglich ist.

Bevorzugt umfasst der Befestigungsabschnitt zumindest ein Befestigungsmittel, welches zumindest eines der Entkopplungselemente an der Basis befestigt. Typischerweise werden entweder lösbare Befestigungsmittel, wie beispielsweise eine Steckbefestigung oder eine Schraubbefestigung, verwendet, wodurch die Stimulierungseinrichtung einfach austauschbar sind und somit eine Wartung der Stimulierungseinrichtung bzw. der Prothesenvorrichtung einfach durchführbar ist.

Insbesondere weist der Prothesenschaft für jeden Vibrationserzeuger eine eigene Prothesenöffnung auf. Damit können voneinander beabstandete Nervenzellenenden stimuliert werden, ohne dass sich die Vibrationserzeuger gegenseitig beeinflussen. Weiters bleibt der Prothesenschaft trotz mehrerer Prothesenöffnungen formstabil und behält eine ausreichend hohe Festigkeit.

Vorteilhaft ist zwischen der zumindest einen Prothesenöffnung und dem zumindest eine Vibrationserzeuger eine Dichteinlage, beispielsweise aus einem Silikonmaterial, angeordnet. Damit kann verhindert werden, dass der Körperteil des Anmelders durch die zumindest eine Prothesenöffnung nach aussen gedrückt wird, wodurch Hautirritationen entstehend könnten.

Vorteilhaft ist die Dichteinlage luftdicht und/oder flüssigkeitsdicht ausgebildet. Damit ist es möglich den zumindest einen Vibrationserzeuger gegenüber Körperflüssigkeiten des Anwenders abzuschirmen. Weiters ermöglicht eine luftdichte Dichteinlage das Verwenden eines Vakuum-Prothesenschafts mit dem zumindest einen Vibrationserzeuger, wobei der Vakuum-Prothesenschaft mithilfe eines Unterdrucksystems an die Extremität des Anwenders angeordnet werden kann.

Alternativ oder ergänzend kann auch ein unlösbares Befestigungsmittel, wie beispielsweise eine Klebstoffverbindung als Befestigungsmittel verwendet werden.

Insbesondere umfasst der Befestigungsabschnitt mehrere Befestigungsmittel zum Befestigen jedes einzelne der Entkopplungselemente. Damit kann jedes der Entkopplungselemente separat am Prothesenschaft befestigt werden.

Bevorzugterweise ist eine elektrische Versorgungseinrichtung am Prothesenschaft vorhanden, wobei die elektrische Versorgungseinrichtung mit dem zumindest einen Vibrationserzeuger elektrisch verbunden ist. Typischerweise ist die wie hier beschriebene Versorgungseinrichtung als wiederaufladbare Batterieeinheit bzw. als Akkumulatoreinheit ausgebildet, sodass der zumindest eine Vibrationserzeuger während der Verwendung der Prothesenvorrichtung am Patienten mit Energie versorgbar ist.

Insbesondere weist die elektrische Versorgungseinrichtung einen Ladeanschluss auf, sodass diese mit einer externen Energieversorgung verbindbar ist.

Insbesondere weist die elektrische Versorgungseinrichtung eine Ladekoppeleinheit auf, mit der die elektrische Versorgungseinrichtung mittels einer separaten Ladeeinheit induktiv wiederaufladbar ist. Damit lässt sich die elektrische Versorgungseinrichtung kabellos wiederaufladen. Beispielsweise weist die Ladekoppeleinheit zumindest einen Magneten zum induktiven Laden der elektrische Versorgungseinrichtung auf.

Vorzugsweise ist zwischen der elektrischen Versorgungseinrichtung und dem zumindest einen Vibrationserzeuger das zumindest eine Entkopplungselement angeordnet und mit der elektrischen Versorgungseinrichtung sowie mit dem zumindest einen Vibrationserzeuger elektrisch verbunden. Damit lässt sich die Energie von der Versorgungseinrichtung über das Entkoppelungselement zum zumindest einen Vibrationserzeuger leiten, sodass keine zusätzlichen Versorgungsleitungen für den Vibrationserzeuger benötigt werden.

Bevorzugterweise ist eine Steuereinrichtung zum Steuern des zumindest einen Vibrationserzeugers am Prothesenschaft vorhanden, wobei der zumindest eine Vibrationserzeuger mit der Steuereinrichtung elektrisch verbunden ist. Damit lässt sich der zumindest eine Vibrationserzeuger mit Stimulationsbefehlen, welche typischerweise von der Steuereinrichtung zum zumindest einen Vibrationserzeuger gesendet werden, versorgen. Dabei wird die Steuereinrichtung so am Prothesenschaft angebracht, dass eine möglichst gewichtsausgleichende Position für die Steuereinrichtung und damit ein höherer Tragekomfort für den Prothesenträger geschaffen werden kann.

Insbesondere weist die Steuereinrichtung einen Prozessor auf, wodurch die wie zuvor beschriebenen Steuermodii an der Prothesenvorrichtung ausführbar sind.

Vorzugsweise weist der Prothesenschaft zumindest einen Positionierabschnitt zum Positionieren des zumindest einen Vibrationserzeuger auf, wobei der Positionierabschnitt zumindest eine Prothesenöffnung aufweist. Dadurch lässt sich der zumindest eine Vibrationserzeuger stabil am Prothesenschaft anordnen und gleichzeitig ein entkoppeltes Stimulieren durch die Prothesenöffnung auf die Nervenzellenenden des Anwenders bzw. Prothesenträgers übertragen

Bevorzugt weist die Prothesenvorrichtung mehrere Stimulierungseinrichtungen auf, wodurch unterschiedliche Nervenzellenenden an der Hautoberfläche stimuliert werden können.

Insbesondere weist jede der mehreren Stimulierungseinrichtungen einen eigenen Befestigungsabschnitt mit einem Befestigungsmittel auf, sodass die jede der mehreren Stimulierungseinrichtungen an der Prothesenvorrichtung, beispielsweise am Prothesenschaft, individuell positioniert werden kann.

Insbesondere jede der mehreren Stimulierungseinrichtungen mit eigenen Versorgungsleitungen mit der Versorgungseinrichtung verbunden, sodass diese unabhängig voneinander versorgbar sind, wobei die Versorgungsleitungen lösbar mit jeder der mehreren Stimulierungseinrichtungen verbunden sind, sodass jede der mehreren Stimulierungseinrichtungen einfach austauschbar ist.

Vorzugsweise weist die Stimulierungseinrichtung zum Stimulieren von Nervenzellenenden zumindest einen Vibrationserzeuger auf, wobei der zumindest eine Vibrationserzeuger mit zumindest einem Entkopplungselement schwingungsfest verbunden ist. Das Entkoppelungselement entkoppelt die Vibrationen bzw. die Schwingungen des Vibrationserzeugers von der Umgebung, beispielsweise von einer Basis, insbesondere von einem Prothesenschaft, und nimmt dabei die Vibrationen bzw. Schwingungen auf, wodurch geräuschbehaftete Vibrationsübertragungen bzw. Schwingungsübertragungen an die Umgebung z.B. an den Prothesenschaft unterbunden werden können. Da keine Vibrationen bzw. Schwingungen übertragen werden, liegt ein schwingungsfester Zustand vor. In anderen Worten ist damit ein herkömmlicher Vibrationserzeuger, wie beispielsweise ein Vibracall-Element, als geräuschloser Stimulator für Nervenzellenenden verwendbar, welcher bisher widmungsgemäss typischerweise eine Geräuschentwicklung während dem Vibrationsvorgang bzw. während dem Schwingungsvorgang vorsieht bzw. eine brummende Geräuschentwicklung wünscht oder als Nebeneffekt in Kauf nimmt. Der Einsatz von Vibracall-Elementen dient bekanntlich dazu, ein Mobiltelefon als Ganzes zum Vibrieren zu bringen, damit ein Träger auch wenn er keinen Klingelton hört, einen eingehenden Anruf wahrnehmen kann. Damit - im Falle vom Vibracall-Modus ein eingehender Anruf trotzdem registriert wird, wenn das Mobiltelefon nicht unmittelbar am Körper getragen wird, ist es Summton, der durch die Resonanzschwingung des Gehäuses des Mobiltelefons entsteht wünschenswert.

Insbesondere ist die neuartige Stimulierungseinrichtung zum Stimulieren von reinnervierten Nervenzellenenden geeignet. Reinnervierten Nervenzellenenden sind Nervenzellenenden, welche von einem Arzt an eine Hautarealoberfläche verlegt wurden und weisen, abhängig vom jeweiligen Patienten bzw. abhängig vom Operationserfolg, eine hohe taktile Empfindlichkeit auf. Daher lassen sich diese besonders sensitiv mit der Stimulierungseinrichtung stimulieren. Wie bei allen Nerven können diese jedoch auch trainiert werden. Die Erfindung soll auch für solche Trainingszwecke zum Einsatz kommen.

Vorzugsweise ist eine Basis vorhanden, an welcher der zumindest eine Vibrationserzeuger angeordnet ist, wodurch der zumindest eine Vibrationserzeuger örtlich stabil gehalten wird. Mit anderen Worten ermöglicht die Basis eine stabile Anordnung des zumindest einen Vibrationserzeugers, wodurch ein reproduzierbares Stimulieren der Nervenzellenenden erfolgen kann.

Bevorzugt ist der zumindest eine Vibrationserzeuger über das zumindest eine Entkopplungselement mit der Basis lagefest verbunden. Damit werden die Vibrationen bzw. Schwingungen von der Basis entkoppelt. Da keine Übertragung der Vibrationen bzw. Schwingungen auf die Basis erfolgt, kann keine Resonanz zwischen dem zumindest einen Vibrationserzeuger und der Basis entstehen, wodurch der zumindest eine Vibrationserzeuger geräuschlos an der Basis vibrieren bzw. schwingen kann.

Vorteilhaft ist der zumindest eine Vibrationserzeuger einstellbar an der Basis angeordnet. Dabei lässt sich die räumliche Lage des zumindest einen Vibrationserzeuger relativ zu der Basis verändern. Dabei kann der zumindest eine Vibrationserzeuger entlang einer ersten Längsachse an der Basis verstellbar angeordnet sein. Alternativ oder ergänzend kann der zumindest eine Vibrationserzeuger um ein Befestigungsmittel drehbar an der Basis angeordnet sein. Damit lässt sich der zumindest eine Vibrationserzeuger individuell auf einzelne Nervenzellenenden einstellen, wodurch das Stimulieren der Nervenzellenenden weiter verbessert werden kann.

Vorteilhaft weist der zumindest eine Vibrationserzeuger zumindest abschnittsweise eine Vibrationsisolation aus einem elastischen Material, wie ein Gummimaterial, auf. Diese Vibrationsisolation verhindert, dass der zumindest eine Vibrationserzeuger schallübertragend mit der Basis wechselwirkt. Damit kann eine zusätzliche Schallentkopplung zur Basis realisiert werden.

Bevorzugt ist zumindest ein weiteres Entkopplungselement vorhanden, wodurch die Entkopplung der Vibrationen bzw. Schwingungen verbessert wird.

Bevorzugt ist das zumindest eine weitere Entkopplungselement mit dem zumindest einen Vibrationserzeuger verbunden. Typischerweise ist das weitere Entkopplungselement ebenfalls zwischen dem zumindest einen Vibrationserzeuger und der Basis angeordnet, wodurch der zumindest eine Vibrationserzeuger stabiler an der Basis angeordnet ist und/oder der zumindest eine Vibrationserzeuger symmetrisch gehalten werden kann.

Vorzugsweise ist das zumindest eine Entkoppelungselement ein Federelement. Damit lässt sich die Vibration bzw. Schwingung des zumindest einen Vibrationserzeugers gegenüber der Basis einfach dämpfen, sodass die Geräuschentwicklung beim Vibrieren bzw. Schwingen unterdrückt wird, ohne dabei gleichzeitig Vibrationsenergie zu vernichten. Als Federelement wird typischerweise ein Draht, wie beispielsweise ein Stahldraht verwendet, wobei der Draht eine ausreichend hohe Festigkeit bzw. Steifigkeit aufweist.

Insbesondere ist das Federelement stabförmig oder spiralförmig ausgebildet, sodass das Federelement einfach an der Basis befestigbar ist und/oder ein verbessertes Entkoppeln beim Vibrieren bzw. Schwingen ermöglicht.

Bevorzugt sind das zumindest eine Entkoppelungselement und das zumindest eine weitere Entkoppelungselement jeweils ein Federelement. Damit ist die Geräuschentwicklung beim Vibrieren bzw. Schwingen vermeidbar, das eine verbesserte Entkopplung realisierbar ist.

Bevorzugterweise ist zumindest das zumindest eine Entkopplungselement elektrisch leitend, wodurch das zumindest eine Entkopplungselement als elektrischer Versorgungsanschluss für den zumindest einen Vibrationserzeuger verwendbar ist.

Insbesondere sind das zumindest eine Entkopplungselement sowie das zumindest eine weitere Entkopplungselement elektrisch leitend, sodass diese als elektrische Versorgungsanschlüsse für den zumindest einen Vibrationserzeuger verwendbar sind. Damit sind keine zusätzlichen elektrischen Versorgungsanschlüsse am zumindest einen Vibrationserzeuger notwendig, wodurch sich damit die Einsatzdauer des zumindest einen Vibrationserzeuger signifikant verlängert. Typischerweise sind die Federelemente Klaviersaiten, da diese einfach mit dem zumindest einen Vibrationserzeuger verbindbar sind, das Vibrieren bzw. Schwingen gut entkoppeln und elektrisch leitend sind.

Vorzugsweise ist eine Versorgungseinrichtung vorhanden, wodurch der zumindest eine Vibrationserzeuger einfach mit elektrischer Energie versorgbar ist. Typischerweise weist die Versorgungseinrichtung eine wiederaufladbare Batterieeinheit bzw. Akkumulatoreinheit und einen Ladeanschluss auf, sodass die wiederaufladbare Batterieeinheit bzw. Akkumulatoreinheit mit einem externen Energiespeicher, bspw. ein Versorgungsspannungsnetz, verbindbar ist und wieder aufgeladen werden kann.

Alternativ oder ergänzend ist eine Steuerungseinrichtung vorhanden, wodurch der zumindest eine Vibrationserzeuger angesteuert werden kann. Typischerweise beinhaltet die Steuereinrichtung einen Prozessor mit mehreren Steuerprogrammen, wodurch der zumindest eine Vibrationserzeuger systematisch ansteuerbar ist.

Insbesondere beinhalten die Steuerprogramme Steuermodii mit unterschiedlichen Steuerbefehlen, die abhängig vom jeweiligen Steuermodus abgerufen werden. Damit lassen sich unterschiedliche Bewegungsabläufe, wie beispielsweise eine Laufbewegung, Gehbewegung, Bergsteigbewegung usw., in der Steuereinrichtung aktivieren.

Bevorzugt umfasst die Steuereinrichtung eine Speichereinheit, worin die Steuerprogramme abgespeichert werden können. Insbesondere ist die Speichereinheit lösbar in der Steuereinrichtung angeordnet, sodass diese austauschbar bzw. die Speicherkapazität erweiterbar ist.

Bevorzugt ist der zumindest eine Vibrationserzeuger mit der Versorgungseinrichtung und/oder mit der Steuerungseinrichtung elektrisch verbunden, wodurch die zuvor genannten Vorteile besonders einfach realisierbar sind.

Bevorzugterweise weist der zumindest eine Vibrationserzeuger ein Exzenterelement oder ein Schwingungselement auf, wodurch sich das Stimulieren der Nervenzellenenden einfach ausführen lässt. Das Exzenterelement oder das Schwingungselement versetzen den zumindest einen Vibrationserzeuger in einen vibrierenden oder schwingenden Zustand. Das Vibrieren wird bei Vibrationserzeugern mit einem Exzenterelement über die wechselnde Zentripetalkraft des Exzenterelements verursacht. Da der Massenschwerpunkt des Exzenterelements an der Antriebswelle des Vibrationserzeugers nicht achsenzentral angeordnet ist, entsteht ein Ungleichgewicht, welche sich auf die Laufruhe des Antriebs auswirkt und den Vibrationserzeuger in Vibration versetzt. Je weiter der Massenschwerpunkt des Exzenterelements von der Drehachse entfernt ist und umso grösser die Masse und die Drehgeschwindigkeit sind, umso grösser wird die Zentripetalkraft und somit die Amplitude der Vibration des Vibrationserzeugers.

Das Vibrieren bzw. Schwingen wird bei Vibrationserzeugern mit einem Schwingungselement durch das Schwingungselement erzeugt, wobei das Schwingungselement über einen kurzen elektromagnetischen Impuls beschleunigt wird und anschliessend über eine Federeinheit wieder zurück in die Ursprungsposition bewegt wird. Das dadurch entstehende Massenträgheitsmoment verursacht eine einfache harmonische Schwingung.

Bevorzugt weist der zumindest eine Vibrationserzeuger einen Antriebsmotor zum rotatorischen Antreiben des Exzenterelement auf, sodass das Exzenterelement in Rotation versetzt werden kann.

Alternativ weist der zumindest eine Vibrationserzeuger einen Antriebsaktuator zum schwingenden Antreiben des Schwingungselements auf, sodass das Schwingungselement in eine harmonische Schwingung versetzt werden kann.

Vorzugsweise weist der zumindest eine Vibrationserzeuger ein Vibrationserzeugergehäuse auf, sodass die Vibrationen bzw. Schwingungen über das Vibrationserzeugergehäuse an die Umgebung bzw. an die Nervenzellenenden weitergegeben werden können.

Bevorzugt ist das Exzenterelement oder das Schwingungselement im Vibrationserzeugergehäuse angeordnet. Damit wird sichergestellt, dass keine freistehenden bewegbaren Bauteile am zumindest einen Vibrationserzeuger vorhanden sind und trotzdem Vibrationen bzw. Schwingungen an die Umgebung bzw. an die Nervenzellenenden abgegeben werden können.

Insbesondere ist das Vibrationserzeugergehäuse als Knopfzelle ausgebildet, in der Antriebsmotor als auch das Exzenterelement bzw. das Schwingungselement angeordnet sind. Damit lässt sich ein einfacher und kompakter Aufbau realisieren, sodass die Schwingungseinrichtung störungsfrei funktioniert.

Bevorzugterweise weist der zumindest eine Vibrationserzeuger eine Stimuliereinheit zum Stimulieren von Nervenzellenenden auf, wodurch die Vibrationen bzw. Schwingungen effektiv in Stimulierungen umgewandelt werden können.

Bevorzugt ist die Stimuliereinheit dornförmig ausgebildet. Damit können die Nervenzellenenden noch punktueller stimuliert werden, wodurch gegebenenfalls eine verbesserte bzw. eine tiefergehende Stimulierung der Nervenzellenenden erfolgt.

Alternativ ist die Stimuliereinheit zylinderförmig oder halbkugelförmig ausgebildet. Damit lässt sich die Stimuliereinheit an unterschiedliche Aufbauten von Nervenzellenenden anpassen, sodass diese effizient stimuliert werden können.

Vorzugsweise ist der zumindest einen Vibrationserzeuger zumindest abschnittsweise mit einer Isolierungsschicht umgeben. Damit wird gewährleistet, dass der zumindest eine Vibrationserzeuger gegenüber Umwelteinflüssen, beispielsweise Feuchtigkeit, geschützt wird und somit die Einsatzdauer des zumindest einen Vibrationserzeugers verlängert wird.

Bevorzugt ist der zumindest einen Vibrationserzeuger vollständig mit der Isolierungsschicht umgeben. Damit ist beispielsweise ein Kurzschluss im bzw. am Vibrationserzeugergehäuse einfach verhinderbar.

Ein weiterer Aspekt der Erfindung betrifft das Bereitstellen eines Vibrationserzeuger mit zumindest einem Exzenterelement oder zumindest einem Schwingungselement (Vibracall*) zum von einer Basis, bevorzugt von einem Prothesenschaft, schwingungsentkoppelten Stimulieren von Nervenzellenenden, insbesondere zum Stimulieren von reinnervierten Nervenzellenende bzw. zur Abgabe von Information von reinnervierten Nervenzellenenden. Damit lässt sich eine besonders effiziente Übertragung eines Vibrationsvorgangs bzw. eines Schwingungsvorgangs auf die Nervenzellenden eines Anwenders bzw. eines Patienten übertragen.

Bevorzugt weist der zumindest eine Vibrationserzeuger dabei zumindest ein Entkopplungselement auf, wodurch die Entstehung eines für den Anwender bzw.

Prothesenträger irritierendes Geräusch unterdrückt werden kann und somit der Tragekomfort verbessert wird.

Ein weiterer Aspekt der Erfindung betrifft die Verwendung der hier beschriebenen Stimulierungseinrichtung als Signalerzeuger in einer Kommunikationsvorrichtung. Damit wird ein geräuschloser taktiler Signalerzeuger in einer Kommunikationsvorrichtung geschaffen. Die Signalerzeuger bzw. der zumindest eine Vibrationserzeuger steht beim Vibrieren nicht in Schwingungsresonanz mit der Kommunikationsvorrichtung, wodurch kein "Brummton" von der Kommunikationsvorrichtung selbst erzeugt wird.

Bevorzugt ist die Kommunikationsvorrichtung eine tragbare Kommunikationsvorrichtung, insbesondere ein Mobiltelefon oder ein Tablet. Damit wird zusätzlich zu den zuvor genannten Vorteile, der Benutzer der tragbaren Kommunikationsvorrichtung bei einem eingehenden Anruf oder bei einer eingehenden Nachricht, aufgrund der punktuellen Vibration bzw. punktuellen Wechselwirkung des zumindest einen Vibrationserzeugers der Schwingungseinrichtung am bzw. mit dem Körper des Benutzers oder an einer bzw. mit einer Auflage der Kommunikationsvorrichtung, beispielsweise einer Tischauflagefläche, darauf aufmerksam gemacht. Damit wird insbesondere die Verwendung eines Mobiltelefons oder eines Tablets verbessert, da sich die eingehenden Anrufe bzw. eingehenden Nachrichten mit mehr Diskretion gegenüber Dritte behandelt lassen.

Vorzugsweise weist die Kommunikationsvorrichtung zumindest eine Gehäuseöffnung auf, in welcher der zumindest eine Vibrationserzeuger, wie hier bereits beschrieben, zumindest abschnittsweise angeordnet ist. Dies bewirkt eine verbesserte punktuelle Verständigung des Benutzers, insbesondere eine punktuelle taktile Verständigung des Benutzers.

Insbesondere weist der zumindest eine Vibrationserzeuger ein Vibrationserzeugergehäuse auf, welches insbesondere zylinderförmig ausgebildet ist, wobei die Zylinderflächen des Zylinders zumindest abschnittsweise in der Gehäuseöffnung angeordnet ist (vertikale Anordnung). Damit werden die Vibrationen direkt entlang der Vibrationsrichtung an den Benutzer übertragen.

Insbesondere ist der zumindest eine Vibrationserzeuger mit der Mantelfläche zumindest abschnittsweise in der Gehäuseöffnung angeordnet (horizontale Anordnung). Damit lässt sich der zumindest eine Vibrationserzeuger einfach in der Gehäuseöffnung platzieren.

Alternativ oder ergänzend lassen sich auch andere geometrische Körper als Vibrationserzeugergehäuse verwenden, wie beispielsweise ein quaderförmiger Vibrationserzeuger. Damit lässt sich die punktuelle Verständigung des Benutzers auf den jeweiligen Verwendungszeck optimieren.

Bevorzugterweise weist die Kommunikationsvorrichtung eine Abdichtung zum Abdichten des zumindest einen Vibrationserzeugers von dem Gehäuseöffnung auf. Damit lässt sich die Kommunikationsvorrichtung völlig resonanzfrei am Gehäuse der Kommunikationsvorrichtung anordnen.

Insbesondere besteht die Abdichtung aus einem weichen bzw. elastischen Material. Typischerweise wird dafür eine Silikonmasse verwendet, welche sich gut verarbeiten lässt und ausreichende Dichtungseigenschaften mit sich bringt.

Vorzugsweise weist die Kommunikationsvorrichtung eine Stimulierungseinrichtung mit einem horizontal angeordneten Vibrationserzeuger auf und eine weiter Stimulierungseinrichtung mit einem vertikal angeordneten Vibrationserzeuger auf. Damit können zwei unterschiedliche Vibrationsmodi bzw. Schwingungsmodi in der Kommunikationsvorrichtung verwendet werden.

Bevorzugt sind der horizontal angeordnete Vibrationserzeuger und der vertikal angeordnete Vibrationserzeuger jeweils in einer eigenen Gehäuseöffnung in der Kommunikationsvorrichtung angeordnet. Damit können die Vibrationen der jeweiligen Vibrationserzeuger separiert werden.

Bevorzugt weist die Kommunikationsvorrichtung eine Auswahlschalteinheit auf zum Auswählen der jeweiligen Vibrationserzeuger auf. Dabei kann der Anwender mithilfe der Auswahlschalteinheit entweder den horizontal angeordneten Vibrationserzeuger auf und einen vertikal angeordneten Vibrationserzeuger als Signalerzeuger auswählen.

Weitere Vorteile, Merkmale und Einzelheiten der Erfindung ergeben sich aus der nachfolgenden Beschreibung, in der unter Bezugnahme auf die Zeichnungen, Ausführungsbeispiele der Erfindung beschrieben sind.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Es zeigen dabei symbolisch:
- Fig. 1: eine erfindungsgemässe Stimulierungseinrichtung mit einer ersten Ausführungsform eines Vibrationserzeugers in einer Seitenansicht,
- Fig. 2: die Stimulierungseinrichtung gemäss Fig. 1 in einer Aufsicht,
- Fig. 3: die Stimulierungseinrichtung gemäss Fig.1 in einer Seitenansicht an einer Basis,
- Fig. 4: die Stimulierungseinrichtung gemäss Fig.1 mit einer weiteren Ausführungsform des Vibrationserzeugers in einer Seitenansicht,
- Fig.5: eine erfindungsgemässe Prothesenvorrichtung mit einer Stimulierungseinrichtung gemäss Fig.1 in einer perspektivischen Darstellung, und
- Fig. 6: die Prothesenvorrichtung gemäss Fig. 5 mit mehreren Stimulierungseinrichtungen gemäss Fig.1 in einer perspektivischen Darstellung.

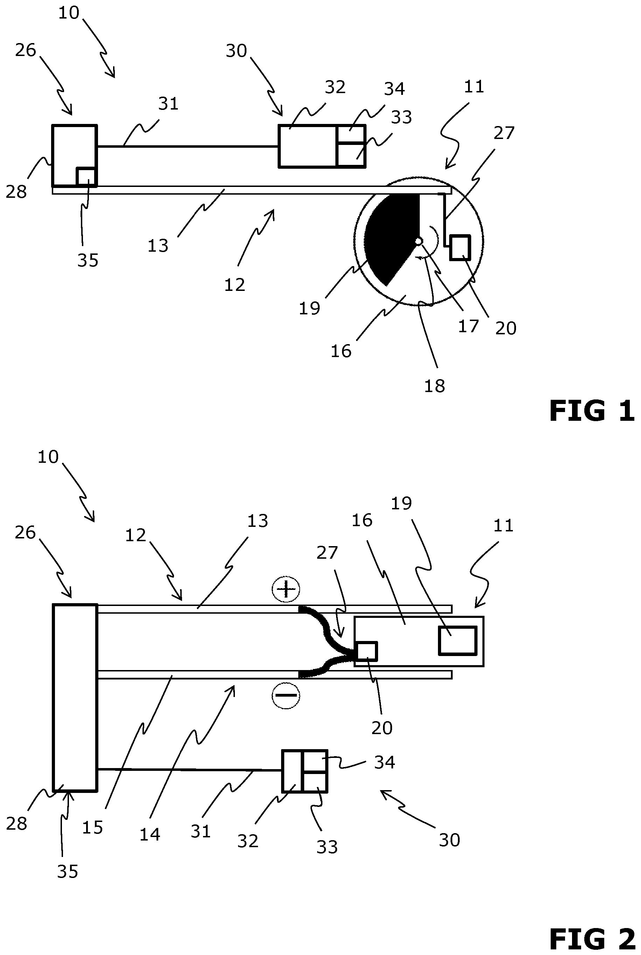

Figur 1 und Figur 2 zeigen die erfindungsgemässe Stimulierungseinrichtung 10 zum Stimulieren von Nervenzellenenden. Die Stimulierungseinrichtung 10 weist einen Vibrationserzeuger 11 mit einem Vibrationserzeugergehäuse 16 auf, das mit einem ersten Federelement 13 bzw. einem zweiten Federelement 15 als Entkopplungselemente 12 bzw. 14 verbunden ist. Das erste Federelement 13 und das zweite Federelement 15 sind stabförmige Federdrähte, welche an gegenüberliegenden Gehäuseseiten des Vibrationserzeugergehäuses 16 angeordnet sind. Dafür sind die Federelemente 13 bzw. 15 jeweils mit einem deren Enden am Vibrationserzeugergehäuse 16 befestigt. Die Federelemente 13 bzw. 15 entkoppeln die Vibrationen bzw. die Schwingungen des Vibrationserzeugers 11 von der Umgebung. Im Vibrationserzeugergehäuse 16 ist ein Exzenterelement 19 angeordnet. Das Exzenterelement 19 ist in Antriebsrichtung 18 drehbar bzw. rotierbar an der Antriebswelle 17 angeordnet. Das Vibrieren des Vibrationserzeugers 11 wird mit dem Exzenterelement 19 über die wechselnde Zentripetalkraft des Exzenterelements 19 verursacht. Da der Massenschwerpunkt des Exzenterelements 19 an der Antriebswelle 17 des Vibrationserzeugers 11 nicht achsenzentral angeordnet ist, entsteht ein Ungleichgewicht, welche sich auf die Laufruhe des Exzenterelements 19 auswirkt (eine Laufunruhe erzeugt) und den Vibrationserzeuger 11 in Vibration versetzt. Je weiter der Massenschwerpunkt des Exzenterelements 19 von der Drehachse der Antriebswelle 17 entfernt ist und umso grösser die Masse und die Drehgeschwindigkeit des Exzenterelements 19 sind, umso grösser wird die Zentripetalkraft und somit die Amplitude der Vibration des Vibrationserzeugers 11. Der Vibrationserzeugers 11 weist in seinem Vibrationserzeugergehäuse 16 einen Antriebsmotor 20 zum rotatorischen Antreiben des Exzenterelements 19 auf. Der Antriebsmotor 20 ist dafür mit der Antriebswelle 17 verbunden. In der dargestellten Form ist der Vibrationserzeuger 11 als Knopfzelle ausgebildet. Dabei ist die Antriebswelle 17, das Exzenterelement 19 und den Antriebsmotor 20 innerhalb des Vibrationserzeugergehäuse 16 angeordnet, welches zylinderförmig ausgebildet ist.

Die Stimulierungseinrichtung 10 weist eine Versorgungseinrichtung 26 auf. Die Versorgungseinrichtung 26 umfasst eine wiederaufladbare Batterieeinheit bzw. einen Akkumulatoreinheit 28 mit einem Ladeanschluss 35 und ist jeweils mit einem Ende des ersten Federelements 13 und des zweiten Federelements 15 verbunden. Die Federelemente 13 bzw. 15 sind elektrisch leitend und versorgen den Antriebsmotor 20 mit elektrischer Spannung. Dabei dient das elektrisch leitende erste Federelement 13 als Versorgung für den positiven Gleichspannungsanschluss und das elektrisch leitende zweite Federelement 15 als Versorgungsleitung für den negativen Gleichspannungsanschluss des Antriebsmotors 20 (Fig. 2). An den Federelementen 13 bzw. 15 sind Versorgungsleitungen 27 angeordnet, welche den Antriebsmotor 20 mit der Batterieeinheit bzw. dem Akkumulatoreinheit 28 verbinden.

Die Stimulierungseinrichtung 10 weist eine Steuereinrichtung 30 mit einem Prozessor 32 auf. Der Prozessor 32 ist mithilfe von Steuerleitungen 31 mit der Versorgungseinrichtung 26 verbunden. Damit lässt sich der Vibrationserzeuger 11 mit unterschiedlichen Steuerbefehlen aus diversen Steuerprogrammen bzw. Steuermodii ansteuern, sodass unterschiedliche Vibrationen bzw. Schwingungen mit dem Vibrationserzeuger 11 realisiert werden können. Die Steuereinrichtung 30 weist eine Speichereinheit 33 auf, in der die unterschiedlichen Steuerbefehle bzw. Steuermodii gespeichert sind und von der die Steuerbefehle an den Prozessor 32 abgerufen werden können. Die Steuereinrichtung 30 weist eine Schnittstelle 34 auf, mit welcher die Steuereinrichtung 30 der Stimulierungseinrichtung 10 mit einem externen Endgerät, beispielsweise einem Computer, Tablet oder Smartphone, verbindbar ist und Steuerbefehle zum Steuern des Exzenterelements 19 des Vibrationserzeugers 11 austauschbar sind.

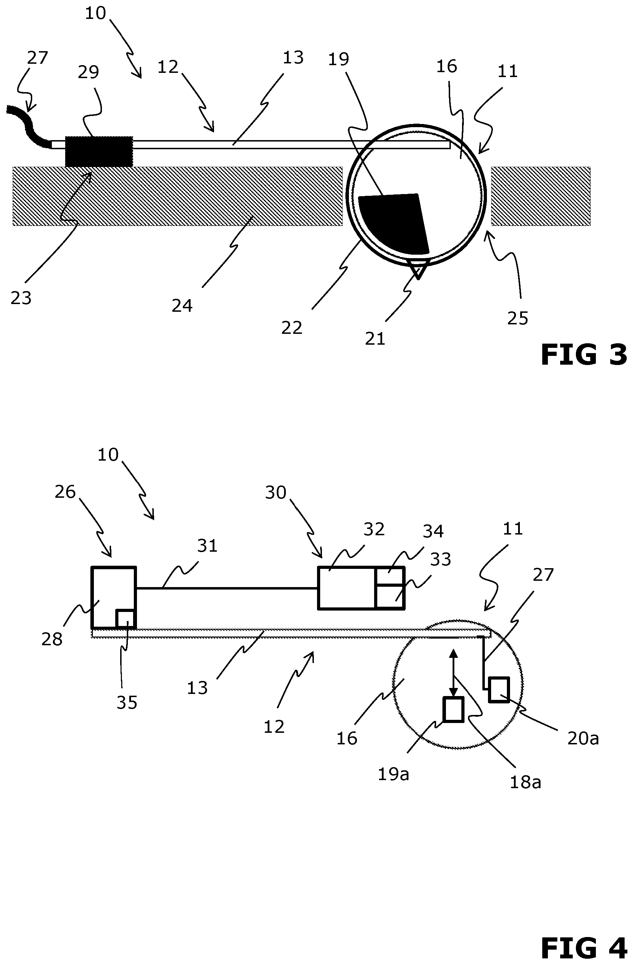

Figur 3 zeigt die zuvor beschriebene erfindungsgemässen Stimulierungseinrichtung 10 und eine Basis 24. Die Stimulierungsvorrichtung 10 bzw. der Vibrationserzeuger 11 ist dabei an der Basis 24 angeordnet. Dafür weist die Stimulierungsvorrichtung 10 an deren Federelemente 13 bzw. 15, welche mit dem Vibrationserzeuger 11 verbunden sind, einen Befestigungsabschnitt 23 auf. Der Befestigungsabschnitt 23 umfasst eine Befestigungsmittel 29, welche die Federelemente 13. bzw. 15 mit der Basis 24 verbinden bzw. an der Basis 24 befestigen. Der Vibrationserzeuger 11 ist einstellbar an der Basis 24 angeordnet. Dabei lässt sich die räumliche Lage des Vibrationserzeuger 11 relativ zu der Basis verändern. Dabei ist der Vibrationserzeuger 11 entlang einer ersten Längsachse an der Basis 24 verstellbar. Zusätzlich ist der Vibrationserzeuger 11 um das Befestigungsmittel 29 drehbar an der Basis 24 angeordnet. Die Basis 24 weist eine Basisöffnung 25 auf. Das Vibrationserzeugergehäuse 16 ist zumindest abschnittsweis in die Basisöffnung 25 eingeführt, sodass das Vibrationserzeugergehäuse 16 die Basisöffnung 25 zumindest teilweise durchdringt. Der Vibrationserzeuger 11 weist, neben dem Exzenterelement 19, zusätzlich eine dornförmige Stimuliereinheit 21 auf, welche am Vibrationserzeugergehäuse 16 angeordnet ist und aus der Basisöffnung 25 hinausragt. Die dornförmige Stimuliereinheit 21 ist dabei an der zum Befestigungsabschnitt 23 gegenüberliegenden Seite der Basis 24 angeordnet. Das Vibrationserzeugergehäuse 16 und die am Vibrationserzeugergehäuse 16 angeordneten jeweiligen Enden des ersten Federelements 13 und des zweiten Federelements 15 sind von einer Isolierungsschicht 22 eingefasst. Die Isolierungsschicht 22 besteht dabei aus Kunstharz. Die Isolierungsschicht 22 kann auch als Vibrationsisolation ausgebildet sein und besteht dabei aus einem elastischen Material, wie ein Gummimaterial. Das erste Federelement 13 und das zweite Federelement 15 sind an deren jeweiligen dem Vibrationserzeugergehäuse 16 gegenüberliegenden Enden bzw. im Bereich des Befestigungsabschnitts 23 jeweils mit Versorgungsleitungen 27 mit der Batterieeinheit bzw. Akkumulatoreinheit 28 verbunden. Alterativ sind die Federelemente 13 bzw. 15 direkt mit der Batterieeinheit bzw. Akkumulatoreinheit 28 verbunden (nicht gezeigt).

Fig.4 zeigt die hier beschriebene Stimulierungseinrichtung 10 mit einem Vibrationserzeuger 11, welcher ein Schwingungselement 19a zum Erzeugen von Vibrationen bzw. Schwingungen im Vibrationserzeuger 11 aufweist. Das Schwingungselement 19a wird über einen kurzen elektromagnetischen Impuls in die Antriebsrichtung 18a beschleunigt und anschliessend wieder zurück in die Ursprungsposition bewegt. Dafür ist im Vibrationserzeugergehäuse 16 des Vibrationserzeugers 11 ein Antriebsaktuator 20a angeordnet, der mit der hier vorliegenden Steuereinrichtung 30 zum Austausch von Steuerbefehlen verbunden ist. Das dadurch entstehende Massenträgheitsmoment verursacht eine einfache harmonische Schwingung. Die mit einem handelsüblichen Vibracall-Element erzielte Vibration erfolgt erst bei einer Schwingungsfrequenz von etwa 200 Hz. Die hier dargestellte Antriebsrichtung 18a ist nur eine von vielen denkbaren Bewegungsrichtungen des Schwingungselements 19a.

Damit ist ein herkömmlicher Vibrationserzeuger 11, wie beispielsweise ein Vibracall, als geräuschloser Stimulator für Nervenzellenenden verwendbar, welcher typischerweise eine Geräuschentwicklung während dem Vibrationsvorgang bzw. dem Schwingungsvorgang vorsieht bzw. eine Geräuschentwicklung wünscht.

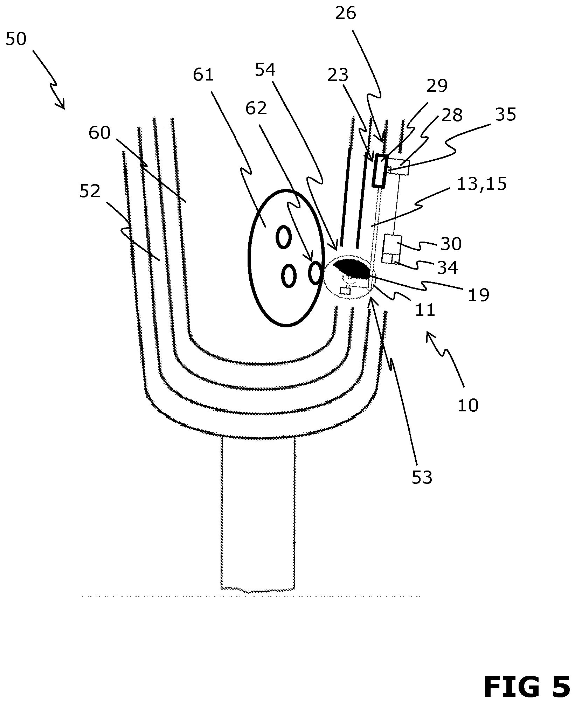

Fig. 5 zeigt die erfindungsgemässe Prothesenvorrichtung 50 mit der hier beschriebenen Stimulierungseinrichtung 10 zum Stimulieren von reinnervierten Nervenzellenenden 62 an einem physionomietypischen Nervenarealabschnitt 61 der Hautoberfläche 60 des Prothesenträgers. Die Stimulierungseinrichtung 10 umfasst den Vibrationserzeuger 11 mit dem hier beschriebenen Exzenterelement 19 und samt den Federelementen 13 bzw. 15, welche als Entkopplungselemente zum Unterbinden der Vibrationen bzw. Schwingungen des Vibrationserzeugers 11 auf den Prothesenschaft 52 dienen. Dabei nimmt der Prothesenschaft 52 die Stellung und Eigenschaften der zuvor beschriebenen Basis ein.

Der Prothesenschaft 52 weist einen Positionierabschnitt 53 zum Positionieren des Vibrationserzeugers 11 an den reinnervierten Nervenzellenenden 62 bzw. an den physionomietypischen Nervenarealabschnitten 61 der Hautoberfläche 60 auf. Im Positionierabschnitt 53 ist eine Prothesenöffnung 54 angeordnet, in welcher der Vibrationserzeugers 11 positioniert wird. Die Stimulierungseinrichtung 10 ist am Prothesenschaft 52 der Prothesenvorrichtung 50 befestigt. Dafür weisen die Federelemente 13 bzw. 15 einen Befestigungsabschnitt 23 mit einem Befestigungsmittel 29 auf. Der Vibrationsgeber 11 ist somit mithilfe der Federelemente 13 bzw. 15 und dem Befestigungsmittel 29 am Prothesenschaft 52 angeordnet. Der Vibrationserzeuger 11 ist einstellbar am Prothesenschaft 52 angeordnet. Dabei lässt sich die räumliche Lage des Vibrationserzeuger 11 relativ zum Prothesenschaft 52 verändern. Dabei ist der Vibrationserzeuger 11 entlang einer ersten Längsachse am Prothesenschaft 52 verstellbar. Zusätzlich ist der Vibrationserzeuger 11 um das Befestigungsmittel 29 drehbar am Prothesenschaft 52 angeordnet.

Am Prothesenschaft 52 ist die Batterieeinheit bzw. die Akkumulatoreinheit 28 samt Ladeanschluss 35 angeordnet und elektrisch mit dem Vibrationserzeuger 11 verbunden. Weiters ist am Prothesenschaft 52 die zuvor beschriebene Steuereinrichtung 30 zum Steuern des Vibrationserzeugers 11 angeordnet, wobei der Vibrationserzeuger 11 mit der Steuereinrichtung 30 elektrisch verbunden ist. Dabei weist die Steuereinrichtung 30 eine Schnittstelle 34 zum Anschliessen eines Endgeräts, wie beispielsweise einen Computer auf. Alternativ weist der Vibrationserzeuger 11 das hier beschriebene Schwingungselement auf (Fig. 4) - nicht gezeigt.

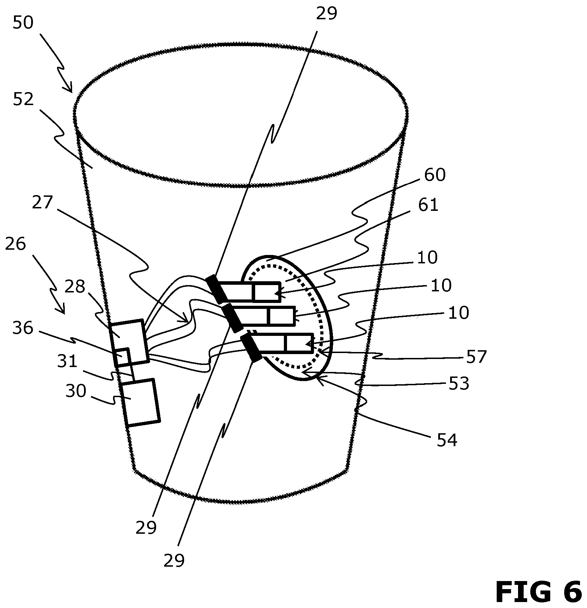

Figur 6 zeigt die zuvor beschriebene Prothesenvorrichtung 50 mit mehreren der hier beschriebenen Stimulierungseinrichtungen 10 zum Stimulieren von reinnervierten Nervenzellenenden bzw. von physionomietypischen Nervenarealabschnitten 61 der Hautoberfläche 60 des Prothesenträgers. Die mehreren Stimulierungseinrichtungen 10 sind jeweils mit einem eigenen Befestigungsmittel 29 am Prothesenschaft 52 befestigt, sodass die Vibrationen bzw. die Schwingungen der einzelnen Vibrationserzeuger der jeweiligen Stimulierungseinrichtung 10 unabhängig voneinander, sowie entkoppelt vom Prothesenschaft 52 dämpfen können. Am Prothesenschaft 52, sind die Steuereinrichtung 30 sowie die Batterieeinheit bzw. Akkumulareinheit 28 angeordnet und mit der Steuerleitung 31 miteinander verbunden. Die mehreren Stimulierungseinrichtungen 10 sind je nach Lage des physionomietypischen Nervenarealabschnitts 61 des Prothesenträgers am Positionierabschnitt 53 individuell angeordnet, mit den jeweiligen Befestigungsmitteln 29 positionierbar und mittel den lösbaren Versorgungsleitungen 27 mit der Batterieeinheit bzw. Akkumulareinheit 28 elektrisch verbunden. Zwischen der Prothesenöffnung 54 und dem Vibrationserzeuger 11 kann eine Dichteinlage 57, beispielsweise aus einem Silikonmaterial, angeordnet sein.

Weiters ist eine elektrische Versorgungseinrichtung 26 am Prothesenschaft 50 vorhanden, wobei die elektrische Versorgungseinrichtung 26 mit den Vibrationserzeugern 11 elektrisch verbunden ist. Dabei weist die elektrische Versorgungseinrichtung 26 eine Ladekoppeleinheit 36 auf, mit der die elektrische Versorgungseinrichtung 26 mittels einer separaten Ladeeinheit induktiv wiederaufladbar ist (nicht gezeigt).

Die hier beschriebenen Stimulierungseinrichtung 10 lässt sich auch als Signalerzeuger in einer Kommunikationsvorrichtung verwenden, wobei diese typischerweise eine tragbare Kommunikationsvorrichtung ist. Dabei ist die Stimulierungseinrichtung 10, wie in den Figuren 1 bis 3 beschrieben, am Gehäuse bzw. an der Basis des Mobiltelefons oder des Tablets angeordnet und wirkt auf die Umgebung, beispielsweise auf die Hautoberfläche des Benutzers. Das Gehäuse der Kommunikationsvorrichtung weist eine Gehäuseöffnung bzw. Basisöffnung auf, in welcher der Vibrationserzeuger der Stimulierungseinrichtung 10 angeordnet ist. Dabei ist der Vibrationserzeuger zumindest abschnittsweise in der Gehäuseöffnung angeordnet, sodass sich der Vibrationserzeuger zumindest abschnittsweise durch die Gehäuseöffnung hindurch erstreckt. Bei dem gezeigten Beispiel, in dem der Vibrationserzeuger zylinderförmig ausgebildet ist, erstreckt sich die Zylinderflächen des Zylinders abschnittsweise durch die Gehäuseöffnung (vertikale Anordnung). Zwischen dem Vibrationserzeuger und der Gehäuseöffnung ist eine weiche bzw. elastische Abdichtung, beispielsweise aus einer Silikonmasse, angeordnet.

Alternativ kann der beschriebene Vibrationserzeuger auch um 90° verdreht zur in den Figuren 1 bis 3 gezeigten Ausführungsform in der Gehäuseöffnung der Kommunikationsvorrichtung angeordnet sein. Dabei erstreckt sich der Vibrationsgeber mit der Mantelfläche abschnittsweise durch die Gehäuseöffnung (horizontale Anordnung). Zwischen dem Vibrationserzeuger und der Gehäuseöffnung ist eine weiche bzw. elastische Abdichtung, beispielsweise aus einer Silikonmasse, angeordnet.

Die Kommunikationsvorrichtung kann weiter eine Stimulierungseinrichtung 10 mit einem horizontal angeordneten Vibrationserzeuger auf und einen vertikal angeordneten Vibrationserzeuger aufweisen, welche jeweils in einer eigenen Gehäuseöffnung in der Kommunikationsvorrichtung angeordnet sind. Die Kommunikationsvorrichtung weist eine Auswahlschalteinheit, einen analogen Schalter oder einen digitalen Schalter, auf zum Auswählen der jeweiligen Vibrationserzeuger auf. Dabei kann der Anwender mithilfe der Auswahlschalteinheit entweder den horizontal angeordneten Vibrationserzeuger auf und einen vertikal angeordneten Vibrationserzeuger als Signalerzeuger auswählen.

### Bezugszeichenliste

- 10: Stimulierungseinrichtung
- 11: Vibrationserzeuger
- 12: Entkopplungselement
- 13: 1. Federelement
- 14: weitere Entkopplungselement
- 15: 2. Federelement
- 16: Vibrationserzeugergehäuse
- 17: Antriebswelle
- 18: Antriebsrichtung von 19
- 18a: Antriebsrichtung von 19a
- 19: Exzenterelement
- 19a: Schwingungselement
- 20: Antriebsmotor
- 20a: Antriebsaktuator
- 21: Stimuliereinheit
- 22: Isolierungsschicht
- 23: Befestigungsabschnitt
- 24: Basis
- 25: Basisöffnung
- 26: Versorgungseinrichtung
- 27: Versorgungsleitung
- 28: Batterieeinheit
- 29: Befestigungsmittel
- 30: Steuerungseinrichtung
- 31: Steuerungsleitung
- 32: Prozessor
- 33: Speichereinheit
- 34: Schnittstelle
- 35: Ladeanschluss
- 36: Ladekoppeleinheit
- 50: Prothesenvorrichtung
- 52: Prothesenschaft
- 53: Positionierabschnitt
- 54: Prothesenöffnung
- 57: Dichteinlage
- 60: Hautoberfläche
- 61: physionomietypischer Nervenarealabschnitt
- 62: reinnervierte Nervenzellenenden

## Patentansprüche

1. Prothesenvorrichtung (50) mit zumindest einer Stimulierungseinrichtung zur Informationsabgabe an Nervenzellenenden, insbesondere zum Stimulieren von Nervenzellenenden, insbesondere physionomietypischen Nervenarealabschnitten (61), mit einem Prothesenschaft (52), wobei die zumindest eine Stimulierungseinrichtung (10) zumindest einen Vibrationserzeuger (11) umfasst, **dadurch gekennzeichnet, dass** der zumindest eine Vibrationserzeuger (11) über zumindest ein Entkopplungselement (12, 14) mit dem Prothesenschaft (52) lagefest verbunden ist.

2. Prothesenvorrichtung (50) nach Anspruch 1, **dadurch gekennzeichnet, dass** das zumindest eine Entkopplungselement (12, 14) einen Befestigungsabschnitt (23) zum Befestigen des zumindest einen Entkopplungselements (12, 14) an dem Prothesenschaft (52) aufweist.

3. Prothesenvorrichtung (50) nach Anspruch 1 oder 2, **dadurch gekennzeichnet, dass** eine elektrische Versorgungseinrichtung (26) am Prothesenschaft (52) vorhanden ist, wobei die elektrische Versorgungseinrichtung (26) mit der Stimulierungseinrichtung (10), insbesondere mit dem zumindest einen Vibrationserzeuger (11), elektrisch verbunden ist.

4. Prothesenvorrichtung (50) nach Anspruch 3, **dadurch gekennzeichnet, dass** zwischen der elektrischen Versorgungseinrichtung (26) und dem zumindest einen Vibrationserzeuger (11) das zumindest eine Entkopplungselement (12, 14) angeordnet ist und mit der elektrischen Versorgungseinrichtung (26) sowie mit dem zumindest einen Vibrationserzeuger (11) elektrisch verbunden ist.

5. Prothesenvorrichtung (50) nach einem der Ansprüche 1 bis 4, **dadurch gekennzeichnet, dass** eine Steuereinrichtung (30) zum Steuern des zumindest einen Vibrationserzeugers (11) am Prothesenschaft (52) vorhanden ist, wobei der zumindest eine Vibrationserzeuger (11) mit der Steuereinrichtung (30) elektrisch verbunden ist.

6. Prothesenvorrichtung (50) nach einem der Ansprüche 1 bis 5, **dadurch gekennzeichnet, dass** der Prothesenschaft (52) zumindest einen Positionierabschnitt (53) zum Positionieren des zumindest einen Vibrationserzeuger (11) aufweist, wobei der Positionierabschnitt (53) zumindest eine Prothesenöffnung (54) aufweist.

7. Prothesenvorrichtung (50) nach einem der Ansprüche 1 bis 6, **dadurch gekennzeichnet, dass** der zumindest eine Vibrationserzeuger (11) mit dem zumindest einem Entkopplungselement (12) schwingungsfest verbunden ist.

8. Prothesenvorrichtung (50) nach einem der Ansprüche 1 bis 7, **dadurch gekennzeichnet, dass** zumindest ein weiteres Entkopplungselement (14) vorhanden ist, welches bevorzugt mit dem zumindest einen Vibrationserzeuger (11) verbunden ist, und bevorzugt das zumindest eine Entkoppelungselement (12) und weiter bevorzugt das zumindest eine weitere Entkoppelungselement (14) ein Federelement (13, 15) ist.

9. Prothesenvorrichtung (50) nach einem der Ansprüche 1 bis 8, **dadurch gekennzeichnet, dass** zumindest das zumindest eine Entkopplungselement (12) elektrisch leitend ist.

10. Prothesenvorrichtung (50) nach einem der Ansprüche 1 bis 9, **dadurch gekennzeichnet, dass** eine Versorgungseinrichtung (26) und/oder Steuerungseinrichtung (30) vorhanden ist, wobei bevorzugt zumindest der zumindest eine Vibrationserzeuger (11) mit der Versorgungseinrichtung (26) und/oder mit der Steuerungseinrichtung (30) elektrisch verbunden ist.

11. Prothesenvorrichtung (50) nach einem der Ansprüche 1 bis 10, **dadurch gekennzeichnet, dass** der zumindest eine Vibrationserzeuger (11) ein Exzenterelement (19) oder ein Schwingungselement (19a) aufweist, bevorzugt einen Antriebsmotor (20) zum rotatorischen Antreiben des Exzenterelement (19) aufweist oder einen Antriebsaktuator (20a) zum schwingenden Antreiben des Schwingungselements (19a) aufweist.

12. Prothesenvorrichtung (50) nach einem der Ansprüche 1 bis 11, **dadurch gekennzeichnet, dass** der zumindest eine Vibrationserzeuger (11) ein Vibrationserzeugergehäuse (16) aufweist, wobei das Exzenterelement (19) oder das Schwingungselement (19a) bevorzugt im Vibrationserzeugergehäuse (16) angeordnet ist.

13. Prothesenvorrichtung (50) nach einem der Ansprüche 1 bis 12, **dadurch gekennzeichnet, dass** der zumindest eine Vibrationserzeuger (11) eine Stimuliereinheit (21) zum Stimulieren von Nervenzellenenden aufweist, wobei die Stimuliereinheit (21) bevorzugt dornförmig ausgebildet ist.

14. Prothesenvorrichtung (50) nach einem der Ansprüche 1 bis 13, **dadurch gekennzeichnet, dass** der zumindest einen Vibrationserzeuger (11) zumindest abschnittsweise mit einer Isolierungsschicht (22) umgeben ist.

## Claims

1. A prosthetic device (50) having at least one stimulation device for transmitting information to nerve cell endings, in particular for stimulating nerve cell endings, in particular physiognomy-typical nerve area sections (61), having a prosthetic socket (52), wherein the at least one stimulation device (10) comprises at least one vibration generator (11), **characterized in that** the at least one vibration generator (11) is securely connected in position to the prosthetic socket (52) via at least one decoupling element (12, 14).

2. The prosthetic device (50) according to claim 1, **characterized in that** the at least one decoupling element (12, 14) has a fastening section (23) for fastening the at least one decoupling element (12, 14) to the prosthetic socket (52).

3. The prosthetic device (50) according to claim 1 or 2, **characterized in that** an electrical power supply device (26) is provided on the prosthetic socket (52), wherein the electrical power supply device (26) is electrically connected to the stimulation device (10), in particular to the at least one vibration generator (11).

4. The prosthetic device (50) according to claim 3, **characterized in that** the at least one decoupling element (12, 14) is arranged between the electrical power supply device (26) and the at least one vibration generator (11) and is electrically connected to the electrical power supply device (26) as well as to the at least one vibration generator (11).

5. The prosthetic device (50) according to any one of claims 1 to 4, **characterized in that** a control device (30) for controlling the at least one vibration generator (11) is provided on the prosthetic socket (52), wherein the at least one vibration generator (11) is electrically connected to the control device (30).

6. The prosthetic device (50) according to any one of claims 1 to 5, **characterized in that** the prosthetic socket (52) has at least one positioning section (53) for positioning the at least one vibration generator (11), wherein the positioning section (53) has at least one prosthetic opening (54).

7. The prosthetic device (50) according to any one of claims 1 to 6, **characterized in that** the at least one vibration generator (11) is connected in a vibration-secure manner to the at least one decoupling element (12).

8. The prosthetic device (50) according to any one of claims 1 to 7, **characterized in that** at least one additional decoupling element (14) is provided, which is preferably connected to the at least one vibration generator (11), and preferably the at least one decoupling element (12) and, more preferably, the at least one additional decoupling element (14) is a spring element (13, 15).

9. The prosthetic device (50) according to any one of claims 1 to 8, **characterized in that** at least the at least one decoupling element (12) is electrically conductive.

10. The prosthetic device (50) according to any one of claims 1 to 9, **characterized in that** a power supply device (26) and/or a control device (30) is provided, wherein, preferably, at least the at least one vibration generator (11) is electrically connected to the power supply device (26) and/or to the control device (30).

11. The prosthetic device (50) according to any one of claims 1 to 10, **characterized in that** the at least one vibration generator (11) has an eccentric element (19) or a vibrating element (19a), preferably has a drive motor (20) for rotatably driving the eccentric element (19), or has a drive actuator (20a) for oscillatingly driving the vibrating element (19a).

12. The prosthetic device (50) according to any one of claims 1 to 11, **characterized in that** the at least one vibration generator (11) has a vibration generator housing (16), wherein the eccentric element (19) or the vibrating element (19a) is preferably arranged within the vibration generator housing (16).

13. The prosthetic device (50) according to any one of claims 1 to 12, **characterized in that** the at least one vibration generator (11) has a stimulation unit (21) for stimulating nerve cell endings, wherein the stimulation unit (21) is preferably shaped like a spike.

14. The prosthetic device (50) according to any one of claims 1 to 13, **characterized in that** the at least one vibration generator (11) is surrounded by an insulating layer (22) at least in sections.

## Revendications

1. Dispositif de prothèse (50) comprenant au moins un dispositif de stimulation pour la délivrance d'informations à des terminaisons de cellules nerveuses, en particulier pour la stimulation de terminaisons de cellules nerveuses, en particulier des sections (61) de zones nerveuses typiques sur le plan physiologique, comprenant une tige de prothèse (52), dans lequel l'au moins un dispositif de stimulation (10) comprend au moins un générateur de vibrations (11), **caractérisé en ce que** l'au moins un générateur de vibrations (11) est relié de manière fixe en position à la tige de prothèse (52) par l'intermédiaire d'au moins un élément de découplage (12, 14).

2. Dispositif de prothèse (50) selon la revendication 1, **caractérisé en ce que** l'au moins un élément de découplage (12, 14) comprend une section de fixation (23) pour la fixation de l'au moins un élément de découplage (12, 14) à la tige de prothèse (52).

3. Dispositif de prothèse (50) selon la revendication 1 ou 2, **caractérisé en ce qu'**un dispositif (26) d'alimentation électrique est prévu sur la tige de prothèse (52), dans lequel le dispositif (26) d'alimentation électrique est relié électriquement au dispositif de stimulation (10), en particulier à l'au moins un générateur de vibrations (11).

4. Dispositif de prothèse (50) selon la revendication 3, **caractérisé en ce que** l'au moins un élément de découplage (12, 14) est agencé entre le dispositif (26) d'alimentation électrique et l'au moins un générateur de vibrations (11), et est relié électriquement au dispositif (26) d'alimentation électrique ainsi qu'à l'au moins un générateur de vibrations (11).

5. Dispositif de prothèse (50) selon l'une des revendications 1 à 4, **caractérisé en ce qu'**un dispositif de commande (30) pour la commande de l'au moins un générateur de vibrations (11) est prévu sur la tige de prothèse (52), dans lequel l'au moins un générateur de vibrations (11) est relié électriquement au dispositif de commande (30).

6. Dispositif de prothèse (50) selon l'une des revendications 1 à 5, **caractérisé en ce que** la tige de prothèse (52) comprend au moins une section de positionnement (53) pour le positionnement de l'au moins un générateur de vibrations (11), dans lequel la section de positionnement (53) comprend au moins une ouverture de prothèse (54).

7. Dispositif de prothèse (50) selon l'une des revendications 1 à 6, **caractérisé en ce que** l'au moins un générateur de vibrations (11) est relié à l'au moins un élément de découplage (12) de manière à isoler les vibrations.

8. Dispositif de prothèse (50) selon l'une des revendications 1 à 7, **caractérisé en ce qu'**au moins un autre élément de découplage (14) est prévu, lequel est de préférence relié à l'au moins un générateur de vibrations (11), et **en ce que**, de préférence, l'au moins un élément de découplage (12) et, de préférence encore, l'au moins un autre élément de découplage (14) est un élément de ressort (13, 15).

9. Dispositif de prothèse (50) selon l'une des revendications 1 à 8, **caractérisé en ce qu'**au moins l'au moins un élément de découplage (12) est électriquement conducteur.

10. Dispositif de prothèse (50) selon l'une des revendications 1 à 9, **caractérisé en ce qu'**un dispositif d'alimentation (26) et/ou un dispositif de commande (30) est prévu, dans lequel de préférence au moins l'au moins un générateur de vibrations (11) est relié électriquement au dispositif d'alimentation (26) et/ou au dispositif de commande (30).

11. Dispositif de prothèse (50) selon l'une des revendications 1 à 10, **caractérisé en ce que** l'au moins un générateur de vibrations (11) comprend un élément excentrique (19) ou un élément oscillant (19a), de préférence comprend un moteur d'entraînement (20) pour l'entraînement en rotation de l'élément excentrique (19) ou comprend un actionneur d'entraînement (20a) pour l'entraînement en oscillation de l'élément oscillant (19a).

12. Dispositif de prothèse (50) selon l'une des revendications 1 à 11, **caractérisé en ce que** l'au moins un générateur de vibrations (11) comprend un boîtier (16) de générateur de vibrations, dans lequel l'élément excentrique (19) ou l'élément oscillant (19a) est de préférence agencé dans le boîtier (16) de générateur de vibrations.

13. Dispositif de prothèse (50) selon l'une des revendications 1 à 12, **caractérisé en ce que** l'au moins un générateur de vibrations (11) comprend une unité de stimulation (21) pour la stimulation de terminaisons de cellules nerveuses, dans lequel l'unité de stimulation (21) est réalisée de préférence sous la forme d'une pointe.

14. Dispositif de prothèse (50) selon l'une des revendications 1 à 13, **caractérisé en ce que** l'au moins un générateur de vibrations (11) est entouré, au moins par sections, d'une couche d'isolation (22).
